# EUROPEAN PATENT APPLICATION

(11) **EP 4 050 017 A1**
(43) Date of publication of application: **31.08.2022**
(21) Application number: 21158984.1
(22) Date of filing: 24.02.2021
(51) Int. Cl.: C07K 1/30, G01N 33/68

(54) **SEEDED PRECIPITATION OF POLYPEPTIDES**

(71) Applicant: PreOmics GmbH, 82152 Planegg/Martinsried (DE)
(72) Inventor: KULAK, Nils A., 80686 München (DE); JOHANSSON, Sebastian H., 82284 Grafrath (DE); HARTINGER, Katrin, 81249 München (DE)
(74) Representative: Dentons Patent Solutions Rechtsanwaltsgesellschaft mbH

(57) **Abstract**

The present invention relates to a method of processing or fractionating a sample comprising proteins, polypeptides and/or peptides, said method comprising (a) changing the physicochemical conditions of said sample; and (b) performing one or both of the following (i) and (ii): (i) adding solid particulate matter to said sample; and (ii) performing said method in a vessel with a rough surface; wherein steps (a) and (b) can be effected concomitantly or in any order; and wherein said processing or fractionating yields one or more first fractions of proteins, polypeptides and/or peptides as a precipitate on said particulate matter and/or said inhomogeneous surface, and a second fraction of proteins, polypeptides and/or peptides remaining in a supernatant.

## Description

This invention relates to a method of processing or fractionating a sample comprising proteins, polypeptides and/or peptides, said method comprising (a) changing the physicochemical conditions of said sample; and (b) performing one or both of the following (i) and (ii): (i) adding solid particulate matter to said sample; and (ii) performing said method in a vessel with a rough surface; wherein steps (a) and (b) can be effected concomitantly or in any order; and wherein said processing or fractionating yields one or more first fractions of proteins, polypeptides and/or peptides as a precipitate on said particulate matter and/or said inhomogeneous surface, and a second fraction of proteins, polypeptides and/or peptides remaining in a supernatant.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Polypeptide/protein fractionation, separation, or depletion remains a challenge in biopharmaceutical research. For example, one of the major goals in current pharmaceutical research is the identification, detection and quantitation of polypeptide/protein biomarkers out of body fluids such as blood plasma. Because of the very high dynamic range of polypeptides/proteins within bodily fluids, low abundant polypeptides/proteins are often difficult or in many instances impossible to detect. For this purpose, most current methods aim to deplete and remove high abundant polypeptides/proteins such as serum albumin and immunoglobulins by selective binding to proteins which have a higher affinity for the respective protein. For example, Protein-G beads are commonly employed to reduce the quantity of IgG protein in the sample of interest. Furthermore, fractionation methods such as ion-exchange chromatography are also often used to separate polypeptides/proteins and reduce the complexity and dynamic range of the sample.

The concept of selective protein/polypeptide precipitation is well established and was described previously (E. J. Cohn, L. E. Strong, W. L. Hughes, D. J. Mulford, J. N. Ashworth, M. Melin, and H. L. Taylor; Journal of the American Chemical Society 1946 68 (3), 459-475; DOI: 10.1021/ja01207a034). Here the pH, temperature, and ethanol content are utilized to induce a step-wise precipitation of certain polypeptides/proteins in solution in blood plasma. This process remains one of the major processes in large-scale protein enrichment in the pharmaceutical industry to purify certain protein/polypeptides species. This process however requires large amounts of starting material to generate a sufficient precipitate that can be filtered or spun down in order to successfully separate the precipitated polypeptides/proteins from the remaining soluble polypeptides/proteins. Furthermore, automatizing filtration or centrifugation remains challenging.

In view of the shortcomings of the state of the art, the technical problem underlying the present invention can be seen in the provision of improved means and methods for sample processing or sample fractionation, in particular of samples comprising polypeptides or in the field of proteomics including research, manufacturing and diagnostics.

This technical problem has been solved by the enclosed claims and as evidenced by the Examples comprised in this disclosure.

Accordingly, the present invention, in a first aspect, relates to a method of processing or fractionating a sample comprising proteins, polypeptides and/or peptides, said method comprising (a) changing the physicochemical conditions of said sample; and (b) performing one or both of the following (i) and (ii): (i) adding solid particulate matter to said sample; and (ii) performing said method in a vessel with a rough surface; wherein steps (a) and (b) can be effected concomitantly or in any order; and wherein said processing or fractionating yields one or more first fractions of proteins, polypeptides and/or peptides as a precipitate on said particulate matter and/or said inhomogeneous surface, and a second fraction of proteins, polypeptides and/or peptides remaining in a supernatant.

Related thereto, the present invention provides a method of processing or fractionating a sample comprising biomolecules, said method comprising (a) changing the physicochemical conditions of said sample; and (b) performing one or both of the following (i) and (ii): (i) adding solid particulate matter to said sample; and (ii) performing said method in a vessel with a rough surface; wherein steps (a) and (b) can be effected concomitantly or in any order.

Preferred biomolecules are biological macromolecules. Preferred biological macromolecules include, in addition to the above disclosed proteins, polypeptides and peptides, nucleic acids, saccharides and lipids.

The term "fractionating" has its art-established meaning. It refers to dividing a mixture of compounds or analytes into at least two separate mixtures, wherein said separate mixtures differ in their relative contents with regard to at least one of said compounds or analytes. In other words, at least one analyte is enriched in at least one fraction as compared to at least one other fraction. Fractionating may lead to at least one analyte being substantially or totally depleted from at least one fraction, but does not have to. In a more general sense, fractionation may lead to a modified or decreased dynamic range or complexity as compared to the original mixture, e.g. the ratio of the amount of most abundant analyte to the amount of the least abundant analyte may be decreased in at least one of the obtained fractions as compared to the original mixture. Such decrease in dynamic range will generally increase the amount of analytes amenable to detection in a subsequent analytical procedures, e.g. mass spectrometry (MS). The term "complexity" as used here refers to the number of analytes, in particular proteins, polypeptides and or peptides comprised in a given fraction.

The terms "protein", "polypeptide" and "peptide" have their art-established meanings as well. In particular, peptides and polypeptides are polycondensates of amino acids, preferably of the proteinogenic L-alpha-amino acids. Herein, such polycondensate is referred to as peptide if it contains 30 or less amino acids, and longer polycondensates are referred to as polypeptides. Preferred peptides are those with a length from 20 to 30 amino acids. Generally, peptides and polypeptides are single chains, which does not exclude the presence of cross-links such as disulfides and/or modifications such as phosphorylation. Proteins on the other, may have a more complex structure in that they may be oligomers of polypeptides and/or comprise non-proteinaceous components such as prosthetic groups. The analysis of such proteins, polypeptides and peptides is of particular interest when it comes to distinguishing healthy from diseased states at a molecular level.

"Physicochemical conditions" embrace both physical parameters such as temperature as well as chemical parameters such as solvent composition. Physicochemical conditions have an influence on the state of analytes in a mixture which state may be expressed in terms of the chemical potential of an analyte. A change in physicochemical conditions is a means to trigger precipitation of one or more analytes in a mixture - to a larger or lesser extent. The changed conditions favor the precipitated state of the analyte.

The term "precipitation" has its art-established meaning, in particular as used in the field of chemistry. It refers to a change of state of the composition of matter under consideration (here proteins, polypeptides, and/or peptides) from dissolved or soluble to insoluble. The insoluble matter may initially form as a suspension which in turn may sediment, for example under the influence of gravity (which may be enhanced by procedures such as centrifugation) to yield a precipitate (insoluble matter in a particular region, generally the bottom of the vessel or container used). In case of centrifugation, the precipitate is commonly referred to as pellet.

Said processing or fractionating yields one or more first fractions of proteins, polypeptides and/or peptides as a precipitate on said particulate matter and/or said inhomogeneous surface, and a second fraction of proteins, polypeptides and/or peptides remaining in a supernatant. Such fractionation is in principle inherent to the method of the first aspect in its broadest definition. This introduces the terms supernatant and precipitate which both have their art-established meanings. After processing in accordance with the method of the first aspect, a part of the proteins, polypeptides and peptides of the original sample has changed its state of matter and is no longer in solution but adheres - as precipitate - to said inhomogeneous surface and/or to said solid particulate matter. The remainder of the analytes is still in solution and constitutes the supernatant.

This introduces the notion of at least one first fraction and a second fraction. The fact that there may be more than one first fraction, i.e., more than one precipitate is owed to the fact that the method of the invention may employ more than one type of particulate matter and/or more than one type of inhomogeneous surface. Preferred is one type of particulate matter or inhomogeneous surface.

Precipitation occurs when a solution is super-saturated, i.e., the solution contains more solute than what can stably be maintained in solution under the conditions given. Yet, a super-saturated state may persist for certain amounts of time. This is because for precipitation to occur, generally a process known as nucleation has to take place. If nucleation does not take place, the super-saturated state may become metastable. "Metastable" refers to a state which is stable in response to slight changes of conditions, but instable when larger changes of conditions occur. Nucleation may be facilitated by seeding. Seeding generally converts a metastable super-saturated state into an instable one which entails precipitation. Seeding, as will become apparent further below, is a key feature of the present invention in mechanistic terms.

Physicochemical conditions have been described as being suitable triggers of precipitation; see the literature cited in the background section above. A listing of preferred physicochemical conditions to be changed in accordance with the invention is subject of a preferred embodiment disclosed further below. The term "harsh" in relation to conditions as used herein refers to physicochemical conditions which are particularly apt to trigger precipitation. Harsh conditions, in extreme cases, may cause precipitation of all or substantially all protein(s), polypeptide(s) and/or peptide(s) from said sample. For that reason, harsh conditions are generally less preferred. As discussed in more detail below, means (b) in accordance with the invention allow to use less harsh conditions including conditions which in the absence of means (b) could not be employed because they would not trigger precipitation at all or to an insufficient extent.

Indeed, the present inventors surprisingly discovered that the effect of a change of physicochemical conditions may be modulated or enhanced - this is expressed in step (b) of the method of the first aspect of the invention. Such modulation or enhancement may, but does not have to, affect different analytes to a different extent. The measures in accordance with step (b) are physical in nature. Their overarching principle is a structured or rough surface. Such structured surface may be embodied as particulate matter to be added to the sample to be processed or fractionated, or as a modification of the surface of the vessel where processing or fractionating takes place.

The structured surface is not particularly limited. It surprisingly turned out that the composition of the particulate matter is irrelevant. Moreover, the structured surface of the vessel may be made of the same material (glass, plastic etc.) as the remainder of the vessel.

The surface of the vessel may be rough throughout or in one or more regions. The size of said one or more regions is macroscopic, wherein as the structural inhomogeneity or roughness in the rough regions is microscopic. For example, the region may have a diameter of 1 to 20 mm such as 2 to 10 mm including 3, 4, 5, 6, 7, 8, or 9 mm. On the other hand, the structural inhomogeneity or roughness is on a nanometer to µm scale, preferably between 0.4 and 100 µm, more preferably between 1 and 60 µm such as 2 to 30 µm including 3, 4, 5, 6, 7, 8, 9, 10, 15, 20 and 25 µm.

Structural inhomogeneity or roughness of a surface may be described of the roughness parameters Ra. Ra is the arithmetical mean deviation from the center line of a profile along the surface under consideration. The dimension of Ra is length; it is usually specified in µm. In a preferred embodiment, the above given preferred values for roughness are Ra values. Roughness may be determined by instruments such as profilometers, optical comparators, interferometers and microscopes. A preferred instrument is a profilometer.

The preferred scale of structural inhomogeneity is also the preferred size of said solid particulate matter.

Without wishing to be bound by a particular theory, it is considered that said solid particulate matter and said rough surface stabilize very small nuclei which always form but would dissolve again in the absence of particles or a rough surface. To explain further, small nuclei have a larger surface to volume ratio as compared to larger nuclei. The free energy of a nucleus depends on both its surface and its volume. In accordance with classical nucleation theory, the surface term is always positive and disfavors nucleation such that the total free energy of nucleation is positive for small nuclei. Only with growing nucleus size, the volume term dominates and eventually nucleation proceeds and precipitation occurs. It is considered that said particulate matter or said rough surface modifies the surface energy of small nuclei such that dominance of the volume term occurs already for very small nuclei.

The method of the invention performs superior when compared to established methods in terms of reducing the dynamic range of analytes in a sample and/or increasing the number of analytes amenable to detection. Reducing dynamic range improves the performance of any downstream analytical method such as mass spectrometry. Analytical methods such as mass spectrometry are inherently characterized by a limited dynamic range within which satisfactory qualitative and quantitative detection is possible. By reducing the dynamic range of the analytes comprised in a sample, e.g. by reducing the relative amount of the most abundant analytes, low abundant analytes which in a sample which has not been processed with the method of the invention would escape detection, become detectable and quantifiable.

The method of the invention furthermore offers a dramatic improvement in processing speed as compared to art-established methods of protein or polypeptide fractionation. While processing over night or for days is common in the art, and also envisaged in accordance with the invention (any processing time between 30 sec and over night (about 12 hrs) or 1 week being preferred), the method of the invention more preferably can be performed on a time scale to be measured in minutes, for example 1 min to 3 hrs, 2 to 30 min, such as 3, 4, 5, 6, 7, 8, 9, 10, 15, 20 or 25 minutes.

A further improvement is the amount of sample sufficient for successful performance of the method. While prior art methods require generally large amounts or even liters of bodily fluids, the present invention works with small sample sizes such as 1 to 1000 µl, 5 to 100 µl such as 10, 20, 30, 40 or 50 µl. In case of bodily fluids with low protein/polypeptide/peptide concentrations such as urine, also larger sample sizes are considered in addition to the ranges and values specified above, such as 1 µl to 100 ml or 10 µl to 50 ml.

As can be seen in the Examples, a proteome under consideration could be analyzed at an unprecedented depth, i.e., the number of proteins amenable to detection by MS was unexpectedly large.

A further advantage of the invention, in particular of the modulating/enhancing effect of an inhomogeneous surface or particulate matter on precipitation is that the physicochemical conditions may be employed in a manner which is less harsh as compared to the art-established procedures. In other words, in the presence of measures in accordance with step (b) of the first aspect, physicochemical conditions may be changed to a lesser extent as compared to the absence of said measures while yielding still satisfactory results in terms of both precipitation and fractionation. Indeed, less strongly precipitating conditions are found to be not only satisfactory, but superior to separate low abundant from high abundant proteins, polypeptides or peptides, e.g. in plasma samples. While strongly precipitating conditions (such as those described in Cohn et al., loc. cit.) generally provide for less selective precipitation of a mixture of protein, polypeptides and/or peptides, less precipitating conditions were surprisingly found to provide for more selective precipitation for seeded precipitation. As demonstrated in the Examples, it was possible to enrich low abundance analytes as compared to highly abundant analytes in the precipitate. Of note, it is envisaged or even preferred to precipitate only a fraction of the total protein(s), polypeptide(s) and/or peptide(s) in said sample. Doing so modifies the dynamic range and the number of detectable protein(s), polypeptide(s) and/or peptide(s) in a favorable manner. Said fraction may be in the range from 0.01 to 50% by weight, more preferably between 0.1 and 20% by weight such as 0.5 to 10% by weight. Yet lower fractions are deliberately envisaged such as 0.0001 to 5% by weight and 0.001 to 1% by weight.

Since, as shown in the Examples, the method of the invention allows the detection of a larger number of protein(s), polypeptide(s) and/or peptide(s) in a given sample (in other words, the proteome is detected with a greater depth), said method provides for novel markers and novel marker combinations for a given state of a cell, tissue or organism ("state" including healthy and diseased), which novel markers in turn provide for improved diagnostics. Given that in particular the detection of protein is of interest in many analytical or diagnostic applications, proteins are the especially preferred analytes.

In a preferred embodiment, said proteins, polypeptides and/or peptides are dissolved in said sample.

In a further preferred embodiment, said sample is a cell lysate or a bodily fluid.

The application of the methods of the invention to a cell lysate extends to what is known in the art as "host cell protein (HCP)" analysis. HCP analysis refers to the monitoring of the purification of a biopharmaceutical product, e.g. an antibody, from the cells, e.g. immortalized B-cells, used for its production/manufacturing. Such purification is generally a stepwise process with host cell proteins being depleted and finally yielding a pharmaceutical grade product. Said host cell proteins include potentially allergenic or otherwise undesirable proteins or polypeptides from the host cell. In other words, HCP analysis may be implemented using the method of the invention.

Bodily fluids include blood, serum, plasma, sputum, nasal swab, urine, vaginal fluid, semen, and cerebrospinal fluid. Cell lysates may be obtained from any cell, tissue or biopsy. Preferably, insoluble parts of said cell lysate are removed, e.g. by centrifugation or filtration prior to performing the method of the invention.

The term "antibody" has its art-established meaning. It embraces all naturally occurring classes and subclasses such as IgG (including IgG1, IgG2, IgG3 and IgG4), IgA, IgM, IgD and IgE as well as camelid antibodies. As used herein, the term "antibody" furthermore embraces fragments (such as Fab) and engineered antigen-binding molecules such as single-chain antibodies, bi- or multispecific antibodies and any antigen-binding molecule with at least three (especially in case of camelid antibodies and their derivatives), preferably six complementarity determining regions (CDRs).

In a further preferred embodiment, step (a), step (b), or both steps (a) and (b) are performed more than once, preferably with the supernatant as defined above. The method of the invention may be performed repeatedly - e.g. once a first precipitate has been removed, the method may be repeated with the supernatant under the same or with modified physicochemical conditions and/or with the same or a different inhomogeneous surface or particulate matter. This allows to further fractionate the supernatant obtained upon the first performance of the method.

In a further preferred embodiment, said physicochemical conditions are one, more or all of: (i) pH value; (ii) temperature; (iii) concentration of at least one organic solvent; (iv) concentration of at least one salt; (v) concentration of at least one surfactant; and (vi) concentration of said protein(s), polypeptide(s) and/or peptide(s).

In a particularly preferred embodiment, the change of pH is towards the isoelectric point of the protein(s), polypeptide(s) and/or peptide(s) to be precipitated on said inhomogeneous surface or solid particulate matter. It is known that proteinaceous molecules are least soluble in the vicinity of their isoelectric point (IEP). Agents which change pH are not particularly limited and include acids and bases.

Preferred acids include inorganic acids such a boric acid, sulfuric acid, nitric acid and hydrogen halides including HF, HC1 and HBr; sulfonic acids such as methane sulfonic acid and benzene sulfonic acid; carboxylic acids such as formic acid, acetic acid and citric acid; halogenated carboxylic acids such as trifluoro acetic acid, chloroacetic acid and fluoroacetic acid.

Preferred bases include metal oxides, metal hydroxides, metal carbonates and metal bicarbonates. Particularly preferred bases are NaOH, KOH, LiOH, CsOH, Mg(OH)₂, Ca(OH)₂, Sr(OH)₂, Ba(OH)₂ and furthermore NH₃, tetramethlyammonium hydroxide, guanidine, butyl lithium and NaH.

In a particularly preferred embodiment, the change in temperature is a decrease. A decrease of temperature generally decreases solubility which affects different proteinaceous analytes to different extents.

In a particularly preferred embodiment, the change in temperature is an increase. Increasing temperature may lead to structural alterations including denaturation which in turn favor precipitation.

In a particularly preferred embodiment, the change of concentration of an organic solvent is an increase or decrease, preferably of an alcohol such as ethanol, methanol, n-propanol, i-propanol; a nitrile such as acetonitrile; a ketone such as acetone; a glycol ether such as 2-methoxy ethanol; an aliphatic hydrocarbon such as hexane; an aromatic hydrocarbon such as benzene; an aldehyde such as formaldehyde; or mixture thereof.

If the sample to be processed is free or essentially free of organic solvents, an increase of concentration is obviously a preferred option. On the other hand, if the sample to undergo precipitation contains an organic solvent, for example because it has undergone pre-processing involving the addition of an organic solvent, decreasing the concentration of said organic solvent may also be a means of fine-tuning and/or optimizing precipitation conditions.

In a particularly preferred embodiment, the change in concentration of a salt is an increase in concentration of said salt, said salt preferably being a kosmotropic salt. "Salting out" using kosmotropic salts is known as a means to trigger precipitation. Preferred kosmotropic salts include ammonium sulfate, potassium phosphate, and salts with a cation selected from , Li+, Zn2+ and Al3+ and an anion selected from carbonate, sulfate and hydrogen phosphate.

It is also envisaged to decrease the concentration of a kosmotropic salt.

In a further particularly preferred embodiment, the change in concentration of a salt is a decrease in concentration of said salt, said salt preferably being a chaotropic salt. Chaotropic salts generally increase solubility. Therefore, a decrease in concentration is preferred. Preferred chaotropic salts are NaCl, MgCl₂, guanidinium hydrochloride, perchlorates such as lithium perchlorate, lithium acetate, Barium salts, thiocyanates, iodides and sodium dodecyl sulfate (SDS).

On the other hand, there may be scenarios where a highly abundant protein is prone to precipitation and presence of large amounts thereof in the precipitate are to be avoided. In such a case, adding a chaotropic salt or increasing its concentration may be desirable.

The different effect of different salts/cations/anions on precipitation is known in the art as the Hofmeister series.

In a particularly preferred embodiment, the change in concentration of a surfactant is an increase in concentration. Adding a surfactant is a means to keep protein, polypeptides and peptides in solution which otherwise have an undesirably high tendency to precipitate. This applies, for example, to transmembrane proteins such as apolipoproteins.

To the extent said sample comprises surfactants or has undergone pre-processing by adding surfactants, it is also envisaged to decrease the concentration of a surfactant. This would generally be a means to trigger precipitation.

Preferred surfactants are sodium lauryl sulfate (SDS), sodium deoxycholic acid (SDC), sodium cholate (SC), 2-[4-(2,4,4-trimethylpentan-2-yl)phenoxy]ethanol (Triton X-100), n-dodecyl-β-D-maltopyranoside (DDM), digitonin, Polyoxyethylene (20) sorbitan monolaurate (polysorbate 20), Polyoxyethylene (80) sorbitan monooleate (polysorbate 80), 3-[(3-Cholamidopropyl)dimethylammonio]-1-propanesulfonate (CHAPS), 3-([3-Cholamidopropyl]dimethylammonio)-2-hydroxy-1-propanesulfonate (CHAPSO), polyethylene glycol nonylphenyl ether (NP-40), n-Octyl beta-D-thioglucopyranoside (OTG), (2R,3S,4S,5R,6R)-2-(hydroxymethyl)-6-octoxyoxane-3,4,5-triol (Octyl glucoside), n-decyl-p-D-maltopyranoside (DM), 3-(4-(1,1-bis(hexyloxy)ethyl)pyridinium-1-yl)propane-1-sulfonate (PPS), sodium 3-((1-(furan-2-yl)undecyloxy)carbonylamino)propane-1-sulfonate (ProteaseMAX), or sodium 3-[(2-methyl-2-undecyl-1,3-dioxolan-4-yl)methoxy]-1-propanesulfonate (RapiGest SF); and mixtures thereof.

Preferred are mild surfactants, i.e., surfactants which do not denaturate proteins, polypeptides and peptides, but solubilize them or portions thereof which are hydrophobic and would aggregate in absence of surfactants. Mild surfactants include deoxycholates such as SDC.

In a particularly preferred embodiment, the change of concentration of said protein(s), polypeptide(s) and/or peptide(s) is an increase. Such increase may be achieved, e.g. by evaporation.

In a particularly preferred embodiment, the change of concentration of said protein(s), polypeptide(s) and/or peptide(s) is a decrease, e.g. by adding solvent such as water or buffer (e.g. PBS). Preferably, said solvent to be added mimics the composition of the sample (disregarding said proteins, polypeptides and peptides).

In a further preferred embodiment, said solid particulate matter is a plurality of microparticles with a diameter between 0.4 and 500 µm, wherein preferably said plurality of microparticles is identical in composition. More preferred size ranges are from 0.5 to 200 µm, 1 to 100 µm, or from 2 to 50 µm such 3, 4, 5, 6, 7, 8, 9, 10, 15, 20 or 25 µm. Particle sizes in a population of particles are generally distributed around a mean. The above figures refer to said mean. Particle sizes can be determined with art-established methods such as scanning electron microscopy (SEM) or static light scattering (SLS).

In a preferred embodiment, the amount of particles to be used is in the range from 0.01 to 100 µg particles per µl sample, more preferably between 0.1 to 10 µg/µl or between 1 and 5 µg/µl such as 2.5 µg/µl.

In a further preferred embodiment, wherein said rough surface is etched or porous.

Generally speaking, and this applies to both said solid particulate matter as well as to said inhomogeneous surface, preferably none of these is derivatized or functionalized in order to provide a surface which would preferentially bind certain analytes or classes of analytes. This is different from many prior art approaches which employ tailored surfaces for each analyte to be enriched or depleted. Indeed, there is no requirement for binding moieties on said surface or said particulate matter. Instead, the effect of said surface and said particulate matter is that of a seed. Seeding is a measure to trigger a change of state of matter which change of matter, in the absence of a seed, would not occur, occur delayed or would require harsher physicochemical conditions to occur.

In a further preferred embodiment, said solid particulate matter consists of or comprises one, more or all of (i) a magnetic or magnetizable composition; (ii) floating particles; and (iii) sedimenting particles. These various types of particles may be used as a single type of particle when working the method of the invention, but also allow for embodiments where more than one type of particulate matter is used, thereby generating a plurality of first fractions or precipitates.

In a further preferred embodiment, said solid particulate matter consists of or comprises polypropylene (PP), polystyrene (PS), polystyrene divinyl benzene (PS-DVB), poly-tetrafluoro ethylene (PTFE), poly-vinyl chloride (PVC), polyoxymethylene (POM), polyethylene (PE) including high-density polyethylene (HDPE) and low-density polyethylene (LDPE), polyamide (PA), polycarbonate (PC), polyethylene terephthalate (PET), polymethyl methacrylate (PMMA), polybutylene terephthalate (PBT), acrylonitrile butadiene styrene (ABS), silica, silanol, ceramics, glass, or metal.

The materials preferred for said solid particulate matter are also preferred vessel materials, said vessel being a vessel with a rough surface according to the present invention.

In a further embodiment, said solid particulate matter carries defined moieties, preferably (i) hydrophobic moieties such as C4, C8, C18 and styrene; and/or (ii) hydrophilic moieties such as hydroxyl, carboxyl, sulfonyl, secondary, tertiary and quaternary amine, preferably hydroxyl, more preferably silanol.

Of note, while such type of functionalized or derivatized particles is envisaged, there is no requirement in accordance with the invention to use them.

The above-disclosed particles are available from various manufacturers and can be prepared by following guidance provided in the literature, e.g. Hench & West, Chem. Rev. 90,33-72 (1990); Rahman & Padavettan, J. Nanomaterials, article ID 132424 (2012); Rao et al., J. Colloid and Interface Sci. 289, 125-131 (2005); and Rahman et al., Colloids and Surface A 294,102-110 (2007).

The following preferred embodiments of the method of the first aspect relate to preferred downstream processing steps which in turn may precede an analytical method further downstream. A preferred downstream analytical method is MS (typically to be performed after one, more or all processing steps (c) to (j) of the following embodiments).

As such, in a preferred embodiment, wherein said processing or fractionating is followed by one or more of the following steps: (c) separating the supernatant from the precipitate; (d) washing the precipitate, and optionally combining the washing solution with said supernatant; and (e) cleaving the protein(s), polypeptide(s) and/or peptide(s) in the supernatant of (c) or (d), preferably with a protease, with a chemical, or mechanically. This embodiment focuses on processing further the supernatant.

In a further preferred embodiment, wherein said processing or fractionating is followed by one or more of the following steps: (f) separating the precipitate from the supernatant; (g) washing the precipitate; (h) solubilizing or resuspending protein(s), polypeptide(s) and/or peptide(s) from said precipitate; and (j) cleaving the protein(s), polypeptide(s) and/or peptide(s) in the eluate obtained in step (h), preferably with a protease, with a chemical, or mechanically. This embodiment focuses on processing further the precipitate. Of note, both this and the preceding preferred embodiment may be used in conjunction.

In a particularly preferred embodiment, wherein use is made of said solid particulate matter, said separating is effected by centrifugation, filtration or by magnetic means.

In a particularly preferred embodiment, wherein use is made of said rough surface, said separating is effected by removing the supernatant such as by pipetting, draining or decanting.

In a further particularly preferred embodiment, said solubilizing or resuspending is performed with a detergent, a solvent such as trifluoro ethanol (TFE), a chaotropic agent such as urea or thiourea, an acid such as trifluoro acetic acid (TFA), a base such as NaOH, or a mixture thereof.

Preferred surfactants are disclosed herein above.

In a further particularly preferred embodiment, (i) said protease is selected from serine proteases, serine carboxy proteases, cysteine proteases, aspartic proteases, metalloproteases and metallocarboxy proteases, preferably from trypsin, chymotrypsin, Lys-C, Lys-N, Asp-N, Glu-C and IdeS; (ii) said chemical is HC1, BrCN, BNPS-skatole, formic acid, hydroxylamine, or 2-nitro-5-thiocyano benzoic acid; or (iii) mechanical cleaving is by means of at least one moving magnet present in said supernatant or eluate, respectively.

Having regard to proteases, the recited classes are EC classes as follows: serine proteases (EC 3.4.21), serine carboxy proteases (EC 3.4.16), cysteine proteases (EC 3.4.22), aspartic proteases (EC 3.4.23), metalloproteases (EC 3.4.24), and metallocarboxy proteases (EC 3.4.17).

Having regard to mechanical cleavage, preferably by means of a moving magnet, reference is made to the present inventors' earlier patent applications WO 2020/002577 and EP patent applications 20174469, 20174484, and 20179317, herewith incorporated by reference in their entirety.

In brief, mechanical cleavage may be effected by a method of cleaving at least one covalent bond in at least one molecule, wherein said method comprises (a) colliding at least one magnetic body with said at least one molecule; and/or (b) triggering collision of at least one non-magnetic particle with said at least one molecule by moving said at least one magnetic body.

Preferred molecules are biomolecules, in particular proteins, polypeptides and peptides.

Said at least one magnetic body may be implemented as a single magnet, i.e., a piece of ferri-, ferro- or paramagnetic material. Said magnetic body preferably performs a fluctuating or oscillating motion. Such motion is preferably triggered by a fluctuating or oscillating magnetic field. The magnetic field may be generated by an electric current which in turn fluctuates or oscillates. The amperage of the current as a function of time is preferably a rectangular function. The electric current preferably flows through a coil surrounding the vessel where the mechanical cleavage is to occur. Said vessel contains the sample to be processed and said at least one magnetic body.

In a further preferred embodiment, fractions, i.e., supernatant and/or precipitate obtained by the method of the invention are subjected to MS, optionally with one, more or all of the intervening processing steps (c) to (j) as disclosed above effected before MS. Suitable MS systems or mass spectrometers include (grouped according to the analyzer):
Time-of-flight (TOF) analyzer such as TIMS-TOF Pro (Bruker) or Synapt XS (Waters), 6230B (Agilent), TripleTOF 6600 (Sciex), LCMS-9030 (Shimadzu);
Orbitrap Analyzer such as Q Exactive Orbitrap series, Orbitrap Tribrid, Orbitrap Exploris series (Thermo Fisher Scientific);
Quadrupole analyzer such as Altis (Thermo Fisher Scientific), Evoq (Bruker), 6400 series (Agilent), QTRAP (Sciex), SCIEX Triple Quad 7500 (Sciex), Xevo series (Waters), LCMS-8060 series (Shimadzu); and
Ion Trap analyzer such as LTQ XL (Thermo Fisher Scientific), amazon series (Bruker), FT-ICR (Fourier Transform-Ion cyclotron resonance) analyzer such as solariX (Bruker) or LTQ FT Ultra (Thermo Fisher Scientific).

Preferably, a liquid chromatography (LC) device, more preferably a micro- or nano-flow LC system, is coupled to the mass spectrometer, preferably online.

In a second aspect, the present invention relates to the use of a vessel with an inhomogeneous surface and/or of solid particulate matter to modulate or enhance precipitation of protein(s), polypeptide(s) and/or peptide(s). Said vessel contains a solution or suspension of said protein(s), polypeptide(s) and/or peptide(s). Said particulate matter is added to said protein(s), polypeptide(s) and/or peptides, generally to a solution or suspension thereof.

Preferred embodiments of the method of the first aspect define, where applicable, preferred embodiments of the use of the second aspect.

In a third aspect, the present invention relates to solid particulate matter with at least one agent adhered thereto, wherein said at least one agent is a trigger of precipitation of protein(s), polypeptide(s) and/or peptide(s).

In a preferred embodiment, (i) said at least one agent is one agent; (ii) said agent is an acid or base; (iii) said agent is a surfactant; and/or (iv) said agent is a salt.

Preferred embodiments of the method of the first aspect define, where applicable, preferred embodiments of the third aspect.

In a fourth aspect, the present invention provides a prefilled reaction vessel, said vessel comprising solid particulate matter in accordance with the third aspect.

Preferred embodiments of the method of the first aspect define, where applicable, preferred embodiments of the fourth aspect.

The Example illustrates the invention.

### Example 1: Seeded precipitation

### Materials and Methods

The precipitation on seeds method in accordance with the invention may be used in conjunction with the iST procedure (Kulak et. al. 2014) utilizing the iST buffers and iST-cartridge from the iST-kit which can be bought from PreOmics GmbH (Planegg, Germany). It adds n additional step prior to the iST process as described below.
Step 1 (additional step): In general, as a first step the precipitation seed - typically magnetic beads with silanol surface chemistry, diameter 3 µm, if not mentioned otherwise - was washed three times with 100 µl ddH₂O ending with the dry seeds. Then, 80 µl of an incubation buffer was added followed by adding 20 µl human blood plasma which corresponsds to roughly 1000 µg of protein content. All components were mixed thoroughly using a vortexer, followed by incubation at room temperature while mixing at 1.000 rpm for 30 minutes. After incubation, the fluid was separated from the seeds using a suitable separation technique, followed by washing the seeds three times with the incubation buffer as above, resulting in dry seeds with precipitated protein on them.
Step 2: Then 50 µl of LYSE-BCT buffer was added followed by heating and shaking for 10 minutes at 95 °C and 1.000 rpm. After cooling to room temperature, 50 µl of resuspended DIGEST was added and incubated at 37 °C for 60 minutes while shaking at 500 rpm.

The digest reaction was quenched adding 100 µl of STOP buffer. The resulting suspension was thoroughly mixed and transferred on an iST-cartridge and then centrifuged for one minute at 3.800 rcf. 200 µl of WASH1 was added followed by centrifugation as just described. This was repeated as before using WASH2. The flow-through was discarded and the cartridge was transferred to a fresh collection tube.

100 µl of ELUTE buffer was added to the cartridge, followed by centrifugation at 3.800 rcf for one minute. The step was repeated once for a total of two elutions gathered in one collection tube.

The eluate was transferred into in an Eppendorf Concentrator under vacuum at 45 °C until completely dry. Typically, 10 µl of the LC-LOAD buffer of the iST-kit were added for resuspending the dried peptides and resuspending properly by shaking and/or vortexing. The concentration of peptides was measured with a NanoDrop instrument.

For analysis of protein and peptide identifications, the solution was injected into a LTQ Orbitrap XL mass spectrometer using a Thermo easy nLC 1200 liquid chromatography system. For the MS measurement, a 45 minute gradient from 5 % buffer A (0.1 % formic acid in water) to 95 % buffer B (0.1 % formic acid in 80 % acetonitrile) was applied for all experiments.

This generic procedure has been implemented in a number of different manners as indicated in the following:
- Experiment 1 (SJ150): The incubation buffer was modified. Sample 1+2: water, sample 3+4: 0,2 x PBS solution, sample 5+6: 0,4 x PBS solution, sample 7+8: 0,6 x PBS solution, sample 9+10: 0,8 x PBS solution, sample 11+12: 1 x PBS solution
- Experiment 2 (SJ154): The incubation buffer was modified. Sample 1+2: water, sample 3+4: 2 % ethanol, sample 5+6: 4 % ethanol, sample 7+8: 6 % ethanol, sample 9+10: 8 % ethanol, sample 11+12: 10 % ethanol
- Experiment 3 (SJ155): The incubation buffer was modified. Sample 1+2: water, sample 3+4: 2 % acetonitrile, sample 5+6: 4 % acetonitrile, sample 7+8: 6 % acetonitrile, sample 9+10: 8 % acetonitrile, sample 11+12: 10 % acetonitrile
- Experiment 4 (SJ156): The incubation buffer was modified. Sample 1+2: pH = 2.4, sample 3+4: pH = 4.2, sample 5+6: pH = 6.0, sample 7+8: pH = 6,92, sample 9+10: pH = 9.0, sample 11+12: pH = 10.94,
- Experiment 5 (SJ164): Incubation buffer at pH 10.94 (as Experiment 4, sample 11+12). Here different seeds have been used. Sample 1+2: C18 magnetic beads, sample 3+4: COOH magnetic beads, sample 5+6: tosyl magnetic beads, Sample 1+2: NH2 magnetic beads, sample 9+10: protein G magnetic beads, sample 11+12 epoxy magnetic beads, sample 13-15: internal control (repeat Experiment 4, sample 11+12)
- Experiment 6 (SJ165): Incubation buffer at pH 10.94 (as Experiment 4, sample 11+12). Here, the effect of transferring the seeds to new flasks after the precipitation step was tested. Sample 1-3: as Experiment 4, sample 11+12 (internal control), sample 4-6: transfer seeds to new flask after precipitation
- Experiment 6 (SJ169): Incubation buffer at pH 10.94 (as Experiment 4, sample 11+12). Here the influence of porosity of seeds was tested. Sample 1-3: 3 zeolith beads were used as seeds, porous material. Sample 4-6: non-porous ceramic beads as seeds
- Experiment 7 (SJ174): Incubation buffer at pH 10.94 (as Experiment 4, sample 11+12). Here the influence of the seed surface was tested. Sample 1-3: glass beads were used as seeds, diameter 100 - 200 µm. Sample 4-6: korund F120 was used as seeds, diameter 90 -125 µm. Sample 7-9: silicium F120 was used as seeds, diameter 90 -125 µm. These materials are typical blasting abrasives.
- Control 1 (SJ150, 13-15): iST-BCT procedure as described above but without any precipitation (step 1). 2 µl human plasma was used as educt.
- Control 2 (SJ130, 1+2; SJ119, 3+4): Step 1 was modified as follows: for sample 1 and 2, 800 µl Blue Sepharose 6 Fast Flow (Merck, GE17-0948-01) was added to a column containing a polyethylene frit. The liquid from the blue Sepharose slurry was pressed through using a syringe. Then, 0.8 ml 0.2 x PBS solution was added and pressed through again. This was repeated twice for a total of three times. 64 µl human plasma was filled up to 100 µl using 0.2 x PBS and added to the washed blue Sepharose beads and incubated for 1 h with 360 ° rotation. Then 400 µl 0.2 x PBS was added and pressed through with syringe. The flow-through was collected and dried in an Eppendorf Concentrator. Half of the dried pellet was then processed as described in Step 2. For sample 3 and 4, 50 µl blue Sepharose beads were washed three times with 100 µl ddH2O, equilibrated 3x with 100 µl equilibration buffer (0.05 m KH₂PO₄, as described by the manufacturer) using quick vortexing (15 sec) and centrifugation (5 sec) and taking off the supernatant. Then 50 µl binding buffer (0.05 m KH₂PO₄, as described by the manufacturer) was added to the relatively dry beads and carefully transferred onto 10 µl human plasma. Incubation was time 10 minutes while shaking at 1.000 rpm for efficient binding. Sample was spun down and the supernatant was transferred to a new reaction flask. 25 µl 2x LYSE-BCT (PreOmics GmbH) was added and Step 2 was performed.
- Control 3 (SJ134): Sample 1 and 2: 100 µl Protein-A magnetic beads (ReSyn Biosciences (Pty) Ltd, South Africa) were washed three times with 300 µl PBS, using magnetic separation to discard the fluid. Then 90 µl binding buffer (PBS, as described by manufacturer) was added to 10 µl human plasma, mixed and added to the washed, dried beads from above. The suspension was incubated for 30 minutes while shaking at 1.000 rpm. After incubation, the supernatant was taken off using magnetic separation and added to 100 µl Protein G beads (ReSyn Biosciences (Pty) Ltd, South Africa), which were washed and dried above. Incubation was performed as above, followed by separation and another round of incubation with Protein G beads. Finally the supernatant was dried in an Eppendorf Concentrator. When dry, Step 2 was performed. Samples 3+ and were treated as above but using Protein A beads (Magtivio) only once.

### Results

### Number of identified proteins

| Protein identific ations | Sample 1 | Sample 2 | Sample 3 | Sample 4 | Sample 5 | Sample 6 | Sample 7 | Sample 8 | Sample 9 | Sample 10 | Sample 11 | Sample 12 | Sample 13 | Sample 14 | Sample 15 |
|---|---|---|---|---|---|---|---|---|---|---|---|---|---|---|---|
| Experiment 1 | 196 | 208 | 202 | 205 | 220 | 214 | 227 | 216 | 222 | 218 | 223 | 213 | N/A | N/A | N/A |
| Experiment 2 | 186 | 204 | 200 | 197 | 193 | 191 | 198 | 194 | 200 | 199 | 195 | 191 | N/A | N/A | N/A |
| Experiment 3 | 200 | 194 | 196 | 201 | 188 | 195 | 197 | 199 | 198 | 197 | 190 | 192 | N/A | N/A | N/A |
| Experiment 4 | 175 | 185 | 176 | 183 | 157 | 166 | 183 | 182 | 226 | 231 | 267 | 279 | N/A | N/A | N/A |
| Experiment 5 | 255 | 251 | 226 | 203 | 267 | 261 | 178 | 231 | 164 | 161 | 276 | 284 | 273 | 284 | 288 |
| Experiment 6 | 261 | 232 | 251 | 260 | 250 | 254 | N/A | N/A | N/A | N/A | N/A | N/A | N/A | N/A | N/A |
| Experiment 7 | 228 | 226 | 241 | 168 | 216 | 213 | 307 | 314 | 300 | 182 | 163 | 177 | N/A | N/A | N/A |

| Protein identifications | Sample 1 | Sample 2 | Sample 3 | Sample 4 |
|---|---|---|---|---|
| Control 1 | 145 | 148 | 137 | |
| Control 2 | 128 | 124 | 137 | 132 |
| Control 3 | 106 | 101 | 127 | 128 |

### Discussion

For all experiments shown a significant increase in protein identifications compared to all controls can be observed. This demonstrates that the precipitation on the seeds in accordance with the invention has the advantages disclosed herein above.

## Claims

1. A method of processing or fractionating a sample comprising proteins, polypeptides and/or peptides, said method comprising
(a) changing the physicochemical conditions of said sample; and
(b) performing one or both of the following (i) and (ii):
(i) adding solid particulate matter to said sample; and
(ii) performing said method in a vessel with a rough surface;
wherein steps (a) and (b) can be effected concomitantly or in any order; and
wherein said processing or fractionating yields one or more first fractions of proteins, polypeptides and/or peptides as a precipitate on said particulate matter and/or said inhomogeneous surface, and a second fraction of proteins, polypeptides and/or peptides remaining in a supernatant.

2. The method of claim 1, wherein said sample is a cell lysate or a bodily fluid.

3. The method of any one of the preceding claims, wherein step (a), step (b), or both steps (a) and (b) are performed more than once, preferably with the supernatant as defined in claim 1.

4. The method of any one of the preceding claims, wherein said physicochemical conditions are one, more or all of:
(i) pH value;
(ii) temperature;
(iii) concentration of at least one organic solvent;
(iv) concentration of at least one salt;
(v) concentration of at least one surfactant; and
(vi) concentration of said protein(s), polypeptide(s) and/or peptide(s).

5. The method of any one of the preceding claims, wherein said solid particulate matter is a plurality of microparticles with a diameter between 0.4 and 500 µm, wherein preferably said plurality of microparticles is identical in composition.

6. The method of any one of the preceding claims, wherein said rough surface is etched or porous.

7. The method of any one of the preceding claims, wherein said solid particulate matter consists of or comprises one, more or all of
(i) a magnetic or magnetizable composition;
(ii) floating particles; and
(iii) sedimenting particles.

8. The method of any one of the preceding claims, wherein said solid particulate matter or said rough surface consists of or comprises polypropylene (PP), polystyrene (PS), polystyrene divinyl benzene (PS-DVB), poly-tetrafluoro ethylene (PTFE), poly-vinyl chloride (PVC), polyoxymethylene (POM), polyethylene (PE) including high-density polyethylene (HDPE) and low-density polyethylene (LDPE), polyamide (PA), polycarbonate (PC), polyethylene terephthalate (PET), polymethyl methacrylate (PMMA), polybutylene terephthalate (PBT), acrylonitrile butadiene styrene (ABS), silica, silanol, ceramics, glass, or metal.

9. The method of any one of claims 1 to 5, 7 or 8, wherein said solid particulate matter carries
(i) hydrophobic moieties such as C4, C8, C18 and styrene; and/or
(ii) hydrophilic moieties such as hydroxyl, carboxyl, sulfonyl, secondary, tertiary and quaternary amine, preferably hydroxyl, more preferably silanol.

10. The method of any one of the preceding claims, wherein said processing or fractionating is followed by one or more of the following steps:
(c) separating the supernatant from the precipitate;
(d) washing the precipitate, and optionally combining the washing solution with said supernatant; and
(e) cleaving the protein(s), polypeptide(s) and/or peptide(s) in the supernatant of (c) or (d), preferably with a protease, with a chemical, or mechanically.

11. The method of any one of the preceding claims, wherein said processing or fractionating is followed by one or more of the following steps:
(f) separating the precipitate from the supernatant;
(g) washing the precipitate;
(h) solubilizing or resuspending protein(s), polypeptide(s) and/or peptide(s) from said precipitate; and
(j) cleaving the protein(s), polypeptide(s) and/or peptide(s) in the eluate obtained in step (h), preferably with a protease, with a chemical, or mechanically.

12. Use of a vessel with an inhomogeneous surface and/or of solid particulate matter to modulate or enhance precipitation of protein(s), polypeptide(s) and/or peptide(s).

13. Solid particulate matter with at least one agent adhered thereto, wherein said at least one agent is a trigger of precipitation of protein(s), polypeptide(s) and/or peptide(s).

14. The solid particulate matter of claim 13, wherein
(i) said at least one agent is one agent;
(ii) said agent is an acid or base;
(iii) said agent is a surfactant; and/or
(iv) said agent is a salt.

15. A prefilled reaction vessel, said vessel comprising solid particulate matter of claim 13 or 14.
